# FASCICULE DE BREVET EUROPEEN

(11) **EP 3 236 901 B1**
(45) Date de publication et mention de la délivrance du brevet: **27.02.2019**
(21) Numéro de dépôt: 15821131.8
(22) Date de dépôt: 15.12.2015
(51) Int. Cl.: A61F 9/02, A63B 33/00, G02C 1/06, G02C 1/00

(54) **LUNETTES**
BRILLE
SPECTACLES

(30) Priorité: 22.12.2014 FR 1463164
(43) Date de publication de la demande: 01.11.2017
(73) Titulaire: Decathlon, 59650 Villeneuve D'ascq (FR)
(72) Inventeur: SAUMUREAU, Damien, 64310 Saint Pee sur Nivelle (FR)
(74) Mandataire: Sayettat, Julien Christian
(86) Numéro de dépôt international: PCT/FR2015/053521
(87) Numéro de publication internationale: WO 2016/102815

(56) Documents cités:
- EP-A1- 0 525 238
- WO-A1-2013/094288
- US-A1- 2009 296 042
- US-A1- 2009 313 746
- US-A1- 2010 064 422
- US-B1- 6 749 299

## Description

L'invention concerne des lunettes, comprenant typiquement une monture équipée d'un dispositif de maintien desdites lunettes sur la tête d'un porteur.

De façon conventionnelle, la monture présente deux cerclages reliés par un pont de nez, et dans chacun desquels est assemblée une unité optique qui comprend une paroi transparente au travers de laquelle la vision s'effectue.

Dans certaines versions, les unités optiques sont assemblées de façon réversible dans les cerclages, notamment à fin de remplacement par exemple en fonction de la vue du porteur, de l'usage et/ou de l'usure de ladite unité.

Le document EP-2 452 724 propose des lunettes dans lesquelles la fixation réversible est réalisée par des moyens géométriques comprenant des languettes interne et externe, la languette interne s'insérant lors de l'assemblage dans un cran présentant une partie saillante vers l'intérieur du cerclage.

Cette réalisation permet un assemblage aisé des unités optiques dans les cerclages tout en assurant une fixation fiable, notamment relativement aux contraintes de déformation que la monture subit lors de l'utilisation des lunettes.

Toutefois, le retrait des unités optiques s'avère difficile à effectuer, notamment en nécessitant un actionnement manuel de la partie saillante qui est située au niveau du pont de nez de la monture.

Le document WO-2013/094288 propose des lunettes dont chaque cerclage peut être équipé d'une unité optique auxiliaire, notamment une unité de protection solaire, ladite unité optique auxiliaire étant positionnée dans ledit cerclage en regard d'une unité optique principale en étant montée dans une gorge périphérique intérieure formée sur le bord avant dudit cerclage.

Toutefois, cette solution ne donne pas entière satisfaction, en ce qu'elle contraint le porteur à engager l'unité optique auxiliaire par l'avant du cerclage, ce qui constitue une ergonomie peu satisfaisante. Par ailleurs, le retrait de l'unité optique s'avère également difficile à effectuer.

L'invention vise à perfectionner l'art antérieur en proposant notamment des lunettes dans lesquelles les unités optiques sont assemblées de façon simple dans les cerclages en assurant une fixation fiable, et ce tout en étant facilement actionnable manuellement en vue de leur retrait.

A cet effet, l'invention propose des lunettes comprenant une monture équipée d'un dispositif de maintien desdites lunettes sur la tête d'un porteur, ladite monture présentant deux cerclages dans lesquels respectivement une unité optique est destinée à être assemblée par l'intermédiaire d'un dispositif de fixation réversible, chacun desdits dispositifs comprenant des moyens géométriques complémentaires prévus sur un bord interne de respectivement le cerclage et l'unité optique, lesdits moyens comprenant une saillie et une gorge dans laquelle ladite saillie est apte à être montée pour assurer une fixation interne de ladite unité optique dans ledit cerclage, chacun desdits dispositifs de fixation réversible comprenant un bouton solidaire d'un bord externe de l'un parmi l'unité optique et le cerclage et une cavité solidaire d'un bord externe de l'autre parmi l'unité optique et le cerclage, ledit bouton étant apte à être inséré dans ladite cavité pour assurer un verrouillage externe de l'unité optique dans le cerclage, chaque cerclage présentant une empreinte débouchant vers l'arrière, chaque unité optique présentant une portée avant qui, à l'état assemblé, est disposée contre une portée complémentaire arrière de ladite empreinte, au moins l'un des bords externes étant déformable réversiblement pour permettre la disposition et le retrait dudit bouton dans ladite cavité lorsque les portées sont disposées l'une contre l'autre.

D'autres particularités et avantages de l'invention apparaîtront dans la description qui suit, faite en référence aux figures annexées, dans lesquelles :
- les figures 1a à 1c sont des représentations partielles de lunettes selon un premier mode de réalisation de l'invention, respectivement en perspective (figure 1a), en vue de dessous (figure 1b) et en vue de l'arrière (figure 1c) ;
- les figures 2a à 2c sont des représentations partielles d'une unité optique des lunettes selon les figures 1, respectivement en vue de l'arrière (figure 2a), en vue du côté interne (figure 2b) et en vue du côté externe (figure 2c) ;
- les figures 3a et 3b sont des représentations partielles montrant un cerclage des lunettes selon les figures 1, respectivement en vue de l'arrière orientée vers l'interne (figure 3a) et en vue de l'arrière orientée vers l'externe (figure 3b) ;
- les figures 4a et 4b sont des représentations partielles de lunettes selon un deuxième mode de réalisation de l'invention, montrant notamment l'assemblage d'une unité optique dans un cerclage, respectivement en vue de dessous (figure 4a) et en perspective (figure 4b), la figure 4c représentant en coupe selon la ligne C-C de la figure 4b un agrandissement de l'assemblage de l'unité optique dans le cerclage.

Dans cette description, les termes de positionnement dans l'espace sont pris en référence à la position des lunettes par rapport au visage d'un porteur ayant revêtu lesdites lunettes. En particulier, le terme « arrière » est relatif à une disposition en regard du visage et le terme « avant » est relatif à une disposition opposée au visage. Par ailleurs, les termes « interne » et « externe » sont relatifs à des dispositions respectivement proche et éloignée du nez du porteur.

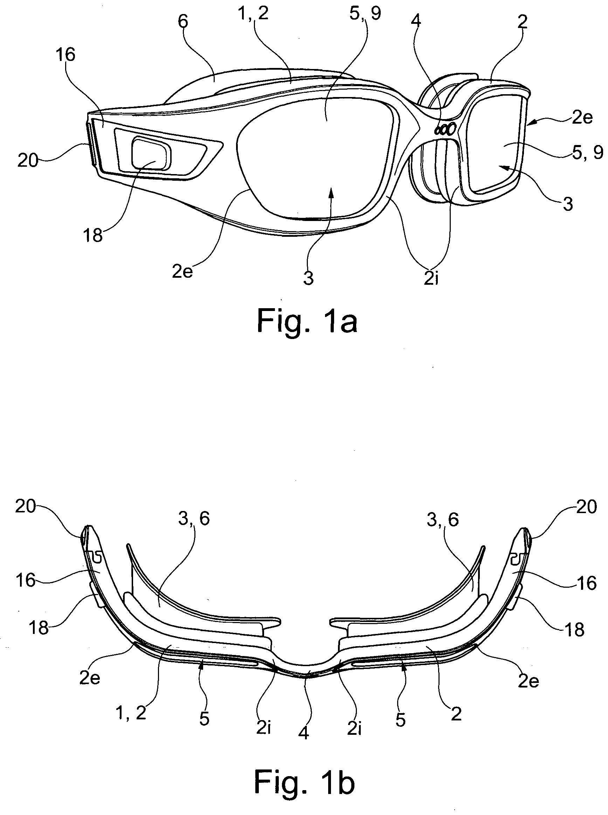

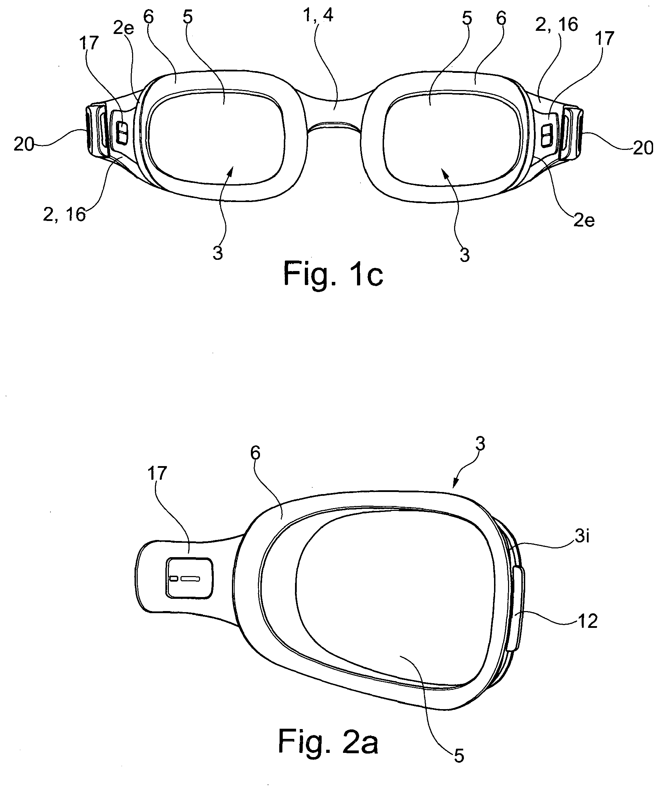

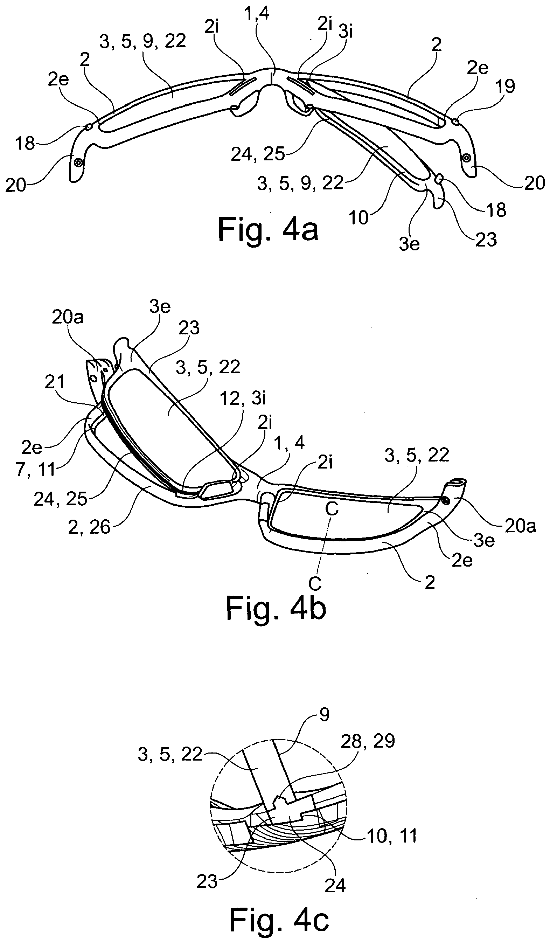

En relation avec ces figures, on décrit ci-dessous des lunettes, notamment destinées à la pratique d'une activité aquatique telle que la natation suivant les figures 1 à 3, ou pour une utilisation loisir ou quotidienne pour la vue selon les figures 4.

Les lunettes comprennent une monture 1 qui présente deux cerclages 2 dans lesquels respectivement une unité optique 3 est destinée à être assemblée de manière réversible, ainsi qu'un pont de nez 4 qui relie les bords internes 2i de chacun desdits cerclages et qui est destiné à être disposé au-dessus du nez d'un porteur lorsqu'il revêt lesdites lunettes.

Les cerclages 2 et les unités optiques 3 peuvent être réalisés en matériau rigide, par exemple à base d'un polymère thermoplastique, afin de permettre leur tenue et leur assemblage réversible par le porteur. Par ailleurs, le pont de nez 4 peut être réalisé en matériau souple, par exemple à base de polymère élastomérique, ledit pont de nez pouvant être surmoulé entre les cerclages 2 pour relier les bords internes 2i en une pièce.

En particulier, le pont de nez 4 est agencé pour bien épouser la forme du nez du porteur et limiter les risques de blessures et/ou de marquage inesthétique sur la peau dudit porteur lorsque les lunettes sont portées longtemps, la déformabilité dudit pont de nez permettant également une bonne adaptation des lunettes à la morphologie du visage dudit porteur.

Chaque unité optique 3 comprend une paroi transparente 5 que le porteur insère dans un cerclage 2 pour assembler ladite unité optique aux lunettes. La paroi transparente 5 est agencée pour que la vision s'effectue au travers d'elle lorsque les lunettes sont revêtues par le porteur, par exemple en étant à base de verre ou d'un matériau polymère thermoplastique transparent. En particulier, les propriétés optiques de la paroi transparente 5 peuvent être agencées pour s'adapter à la vue du porteur afin qu'il puisse sélectionner des unités optiques 3 qui lui conviennent avant de les assembler dans les cerclages 2 de ses lunettes.

Chaque cerclage 2 présente une empreinte 7 débouchant vers l'arrière et au centre de laquelle est formée une ouverture 8. Par ailleurs, la face avant 9 de chaque paroi transparente 5 est disposée dans l'ouverture 8 pour permettre la vision du porteur, ladite face avant étant entourée par une portée avant 10 qui, à l'état assemblé, est disposée en appui avant contre une portée complémentaire 11 de l'empreinte 7.

En particulier, chaque unité optique 3 est destinée à être assemblée dans un cerclage 2 par l'intermédiaire d'un dispositif de fixation réversible, ladite fixation assurant, dans le cas de lunettes de natation, notamment l'étanchéité de l'appui entre les portées 10, 11 afin d'empêcher la pénétration d'eau à l'intérieur de l'unité optique 3.

Chacun des dispositifs comprend des moyens géométriques complémentaires prévus sur un bord interne 2i, 3i de respectivement le cerclage 2 et l'unité optique 3. Sur les figures, les moyens géométriques complémentaires comprennent une saillie 12 et une gorge 13 dans laquelle ladite saillie est apte à être montée pour assurer une fixation interne d'une unité optique 3 dans un cerclage 2.

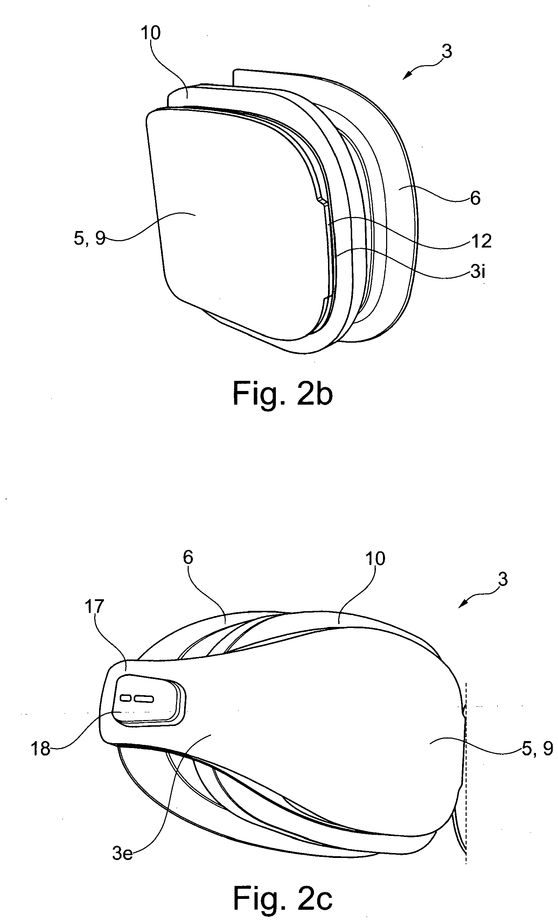

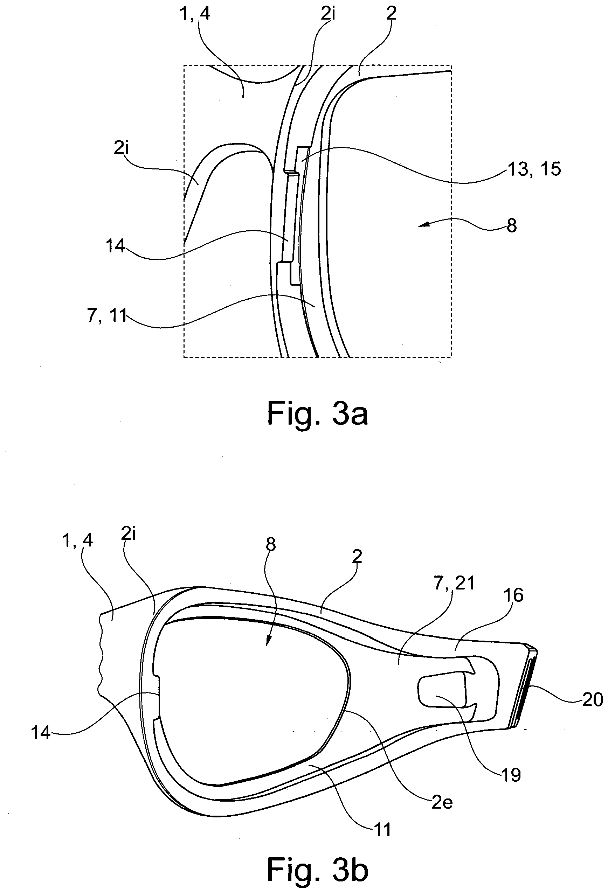

En relation avec les figures 2b et 3a, chaque unité optique 3 présente une saillie 12 qui s'étend intérieurement depuis le bord interne 3i de la paroi transparente 5 en étant disposé en regard vers l'avant de la portée 10. En variante, la saillie 12 peut être formée directement par le bord interne 3i qui est alors agencé pour pouvoir s'engager dans la gorge 13.

Chaque cerclage 2 comprend une gorge 13 qui est formée entre la portée complémentaire 11 et une saillie arrière 14 formée sur l'empreinte 7. Ainsi, une gorge 13 est formée entre une portée complémentaire 11 et une saillie arrière 14 formée à l'intérieur d'un cerclage 2, afin de permettre un bon positionnement de la fixation interne. Par ailleurs, chaque gorge 13 présente un évidement 15 formé dans l'empreinte 7 afin de faciliter l'insertion de la saillie 12 dans la gorge 13.

Pour permettre le retrait des unités optiques 3, notamment pour remplacer lesdites unités optiques en fonction de leur usage et/ou de leur usure, chaque dispositif de fixation réversible comprend en outre un bouton 18 solidaire d'un bord externe 2e, 3e de l'un parmi l'unité optique 3 et le cerclage 2 et une cavité 19 solidaire d'un bord externe 2e, 3e de l'autre parmi l'unité optique 3 et le cerclage 2, ledit bouton étant apte à être inséré dans ladite cavité pour assurer un verrouillage externe de l'unité optique 3 dans le cerclage 2, au moins l'un des bords externes 2e, 3e étant déformable réversiblement pour permettre la disposition et le retrait dudit bouton dans ladite cavité lorsque les portées 10, 11 sont disposées l'une contre l'autre.

Le fait que les moyens de verrouillage/déverrouillage des unités optiques 3 soient situés du côté des bords externes 2e, 3e permet de faciliter l'accès auxdits moyens, et donc l'actionnement manuel desdits moyens en vue du retrait des unités optiques 3. Par ailleurs, l'engagement par l'arrière des unités optiques 3 dans les cerclages 2 procure une ergonomie de déplacement et de déformation qui est satisfaisante tant en verrouillage qu'en déverrouillage.

Dans les modes de réalisation représentés, le bord externe 2e de chaque cerclage 2 s'étend latéralement depuis la portée complémentaire 11 et comprend une extrémité libre équipée d'un moyen 20, 20a pour permettre l'attache d'un dispositif de maintien des lunettes sur la tête du porteur.

Sur les figures, la cavité 19 est formée dans le bord externe 2e du cerclage 2. Par ailleurs, le bouton 18 s'étend sur l'avant du bord externe 3e de l'unité optique 3 et est agencé de manière à être retirable de la cavité 19 par appui manuel sur lui.

Pour assembler une unité optique 3 dans un cerclage 2, le porteur monte d'abord la saillie 12 dans la gorge 13, notamment en inclinant le bord interne 3i de l'unité optique 3 vers le bord interne 2i du cerclage 2, afin d'assurer la fixation interne de ladite unité optique.

Ensuite, le porteur dispose le bouton 18 dans la cavité 19, notamment en rabattant le bord externe 3e de l'unité optique 3 vers le bord externe 2e du cerclage 2 pour déplacer les portées 10, 11 l'une contre l'autre, afin d'assurer le verrouillage externe de ladite unité optique dans ledit cerclage.

De façon avantageuse, la saillie 12 est agencée pour, lors de son montage dans la gorge 13 par inclinaison interne de l'unité optique 3, former une charnière pour le rabattement de la portée avant 10 de ladite unité optique contre la portée arrière du cerclage 2 en vue du verrouillage externe de ladite unité optique.

En outre, les bords externes 2e, 3e sont agencés pour, lors de ce rabattement, assurer la déformation élastique d'un desdits bords externes par mise en appui sur l'autre bord externe 2e, 3e afin de permettre la disposition du bouton 18 dans la cavité 19. En particulier, les bords externes 2e, 3e présentent une longueur suffisante pour autoriser un léger déplacement relatif. Ensuite, le bouton 18 et la cavité 19 étant issus d'une structure en matériau rigide, ils permettent de garantir la fiabilité du verrouillage.

Dans les modes de réalisation représentés, le bord externe 3e de l'unité optique 3 présente une géométrie avant qui est complémentaire de la géométrie arrière du bord externe 2e du cerclage 2. Plus précisément, l'empreinte 7 de chaque cerclage 2 présente une extension latérale 21 qui s'étend sur la géométrie arrière du bord externe 2e, la géométrie avant du bord externe 3e étant, à l'état assemblé, en appui avant sur ladite extension.

En particulier, la cavité 19 et le bouton 18 sont formés respectivement dans l'extension latérale 21 et sur la géométrie avant du bord externe 3e, notamment en étant chacun au niveau de leur extrémité libre. Par ailleurs, le bouton 18 et/ou la cavité 19 peuvent être agencés pour que la disposition dudit bouton dans ladite cavité s'accompagne d'un bruit caractéristique de clipsage, afin d'indiquer au porteur que le verrouillage de l'unité optique 3 dans le cerclage 2 a été correctement effectué.

Ainsi, l'unité optique 3 est montée de façon stable et fiable dans le cerclage 2, notamment en limitant au maximum les risques de retrait intempestif de ladite unité optique, qui peuvent par exemple survenir suite aux contraintes de déformation subies par la monture 1 lors de l'utilisation des lunettes.

Pour retirer une unité optique 3, le porteur doit d'abord déverrouiller extérieurement l'unité optique 3 en retirant le bouton 18 de la cavité 19, par exemple en appuyant manuellement sur ledit bouton et/ou en déformant l'un des bords externes 2e, 3e. Pour effectuer ce déverrouillage, le porteur peut exercer un effort manuel vers l'avant sur le bord externe 2e, notamment au moyen de son index à proximité de l'extrémité libre dudit bord externe, combiné à un effort vers l'arrière sur le bouton 18, notamment par enfoncement au moyen de son pouce.

Ensuite, le porteur doit retirer la saillie 12 de la gorge 13 pour retirer totalement l'unité optique 3 du cerclage 2. Pour ce faire, le porteur peut incliner vers l'arrière le bord externe 3e de l'unité optique 3, et ce en utilisant la charnière formée par la saillie 12 encore disposée dans la gorge 13, afin d'écarter ledit bord externe du bord externe 2e du cerclage 2. Enfin, le porteur n'a plus qu'à effectuer un simple mouvement de traction vers l'extérieur pour retirer la saillie 12 de la gorge 13.

En relation avec les figures 1 à 3, on décrit ci-dessous un première mode de réalisation, notamment pour des lunettes destinées à la pratique d'une activité aquatique telle que la natation.

En particulier, le bord externe 3e et la portée avant 10 de chaque unité optique 3 sont formés directement sur la paroi transparente 5, et l'assemblage de l'unité optique 3 dans un cerclage 2 est agencé pour assurer notamment l'étanchéité de l'appui entre les portées 10, 11, afin d'empêcher la pénétration d'eau à l'intérieur de l'unité optique 3.

Par ailleurs, la paroi transparente 5 de chaque unité optique 3 présente une périphérie arrière qui est équipée d'un joint 6 destiné à épouser le visage du porteur, notamment afin d'empêcher la pénétration d'eau entre son oeil et ladite paroi transparente. En particulier, le joint 6 peut être réalisé en matériau élastomère souple et être agencé pour venir en appui périphérique étanche autour de l'oeil du porteur.

Chaque bord externe 2e, 3e présente en outre une patte 16, 17 qui s'étend vers l'arrière depuis son extrémité libre, ladite patte portant respectivement le bouton 18 et la cavité 19.

En particulier, la patte 16 de chaque cerclage 2 s'étend vers l'arrière depuis la portée complémentaire 11 et comprend une extrémité libre équipée d'une fente 20 pour permettre l'attache d'un dispositif de maintien des lunettes sur la tête du porteur, par exemple une bande élastique de longueur ajustable. En particulier, la fente 20 peut permettre l'insertion directement d'une bande d'attache ou, comme représenté, par l'intermédiaire d'une pièce destinée à être fixée dans la fente 20.

De même, la patte 17 de chaque unité optique 3 s'étend vers l'arrière depuis le bord externe 3e de la paroi transparente 5 en dépassant latéralement du joint 6.

Sur les figures 1 à 3, la cavité 19 est formée latéralement dans la patte 16 du cerclage 2, notamment en débouchant latéralement de ladite patte. Par ailleurs, le bouton 18 s'étend extérieurement depuis la patte 17 de l'unité optique 3 et est agencé de manière à être retirable de la cavité 19 par appui latéral manuel sur lui.

De façon avantageuse, la surface extérieure de la patte 16 peut être revêtue d'un matériau élastomère souple qui recouvre au moins la cavité 19, masquant le bouton 18 lors de sa disposition dans ladite cavité tout en permettant son actionnement manuel, ce qui permet notamment d'assurer l'étanchéité en évitant toute infiltration d'eau au niveau de ladite cavité.

Par ailleurs, au moins l'une des pattes 16, 17 peut être déformable réversiblement pour permettre la disposition et le retrait du bouton 18 dans la cavité 19 lorsque les portées 10, 11 sont disposées l'une contre l'autre.

Ainsi, les pattes 16, 17 peuvent être agencées pour, lors du rabattement de la portée avant 10 de l'unité optique 3 contre la portée arrière 11 du cerclage 2, assurer la déformation élastique d'une desdites pattes par mise en appui sur l'autre patte 16, 17, afin de permettre la disposition du bouton 18 dans la cavité 9. En particulier, les pattes 16, 17 présentent une longueur suffisante pour autoriser un léger déplacement relatif.

En outre, pour retirer le bouton 18 de la cavité 19, le porteur peut appuyer manuellement sur ledit bouton et/ou déformer l'une des pattes 16, 17, par exemple en exerçant avec son index un effort manuel vers l'avant sur l'extrémité libre de la patte 16, combiné à un enfoncement sur le bouton 18 avec son pouce.

En relation avec les figures 4, on décrit ci-dessous un deuxième mode de réalisation, notamment pour des lunettes de vue et/ou de soleil. En particulier, chaque bord extérieur 2e du cerclage présente une extension 20a qui s'étend vers l'arrière depuis son extrémité libre et qui est agencée pour permettre le montage en rotation d'une branche pliable pour assurer le maintien des lunettes sur la tête du porteur.

Chaque unité optique 3 comprend un verre 22 dans lequel est formée la paroi transparente 5 et dont la périphérie est équipée d'un adaptateur 23. En particulier, l'adaptateur 23 est réalisé en matériau polymérique en entourant la périphérie du verre 22, ledit adaptateur pouvant être associé irréversiblement, par exemple par surmoulage.

En variante, l'association peut être réversible, par exemple en prévoyant qu'un adaptateur puisse permettre le montage de plusieurs types de verre 22, différant par leur fonction et/ou par leur épaisseur.

En particulier, l'adaptateur 23 et le verre 22 présentent des moyens géométriques complémentaires d'association. En relation avec la figure 4c, chaque verre 22 et chaque adaptateur 23 présentent respectivement une rainure 28 et une nervure complémentaire 29 d'association qui s'étendent respectivement le long de la périphérie extérieure du verre 22 et le long de la périphérie intérieure de l'adaptateur 23. En variante, la périphérie du verre 22 peut être équipée de la nervure, la périphérie de l'adaptateur 23 présentant alors la rainure.

Le bord externe 3e et la portée avant 10 sont formés sur l'adaptateur 23 qui est plus facilement optimisable que le verre 22 pour garantir la fonction d'assemblage de l'unité optique 3 dans le cerclage 2. En particulier, la périphérie extérieure de l'adaptateur 23 présente une marche 24 sur laquelle est formée la portée avant 10, ladite marche étant en appui sur la portée arrière 11, lesdites portées pouvant s'étendre sur tout ou partie des périphéries respectivement de l'adaptateur 23 et du cerclage 2.

## Revendications

1. Lunettes comprenant une monture (1) équipée d'un dispositif de maintien desdites lunettes sur la tête d'un porteur, ladite monture présentant deux cerclages (2) dans lesquels respectivement une unité optique (3) est destinée à être assemblée par l'intermédiaire d'un dispositif de fixation réversible, chacun desdits dispositifs comprenant des moyens géométriques complémentaires prévus sur un bord interne (2i, 3i) de respectivement le cerclage (2) et l'unité optique (3), lesdits moyens comprenant une saillie (12) et une gorge (13) dans laquelle ladite saillie est apte à être montée pour assurer une fixation interne de ladite unité optique dans ledit cerclage, chacun desdits dispositifs de fixation réversible comprenant un bouton (18) solidaire d'un bord externe (2e, 3e) de l'un parmi l'unité optique (3) et le cerclage (2) et une cavité (19) solidaire d'un bord externe (2e, 3e) de l'autre parmi l'unité optique (3) et le cerclage (2), ledit bouton étant apte à être inséré dans ladite cavité pour assurer un verrouillage externe de l'unité optique (3) dans le cerclage (2), lesdites lunettes étant **caractérisées en ce que** chaque cerclage (2) présente une empreinte (7) débouchant vers l'arrière, chaque unité optique (3) présentant une portée avant (10) qui, à l'état assemblé, est disposée contre une portée complémentaire arrière (11) de ladite empreinte, au moins l'un des bords externes (2e, 3e) étant déformable réversiblement pour permettre la disposition et le retrait dudit bouton dans ladite cavité lorsque les portées (10, 11) sont disposées l'une contre l'autre.

2. Lunettes selon la revendication 1, **caractérisées en ce qu'**au moins un bord externe (2e, 3e) est équipé d'une patte (16, 17) s'étendant vers l'arrière, ladite patte portant le bouton (18) ou la cavité (19).

3. Lunettes selon l'une des revendications 1 ou 2, **caractérisées en ce que** les moyens géométriques complémentaires sont agencés pour que la saillie (12) puisse être d'abord montée dans la gorge (13), le déplacement ultérieur des portées (10, 11) l'une contre l'autre permettant le verrouillage externe dudit montage par disposition du bouton (18) dans la cavité (19).

4. Lunettes selon la revendication 3, **caractérisées en ce que** la saillie (12) est agencée pour être montée dans la gorge (13) par inclinaison interne de l'unité optique (3) en formant une charnière pour le rabattement de la portée avant (10) de ladite unité optique contre la portée arrière (11) du cerclage (2) en vue du verrouillage externe de ladite unité optique.

5. Lunettes selon la revendication 4, **caractérisées en ce que** les bords externes (2e, 3e) sont agencés pour, lors du rabattement, assurer la déformation élastique d'un desdits bords par mise en appui sur l'autre desdits bords afin de permettre la disposition du bouton (18) dans la cavité (19).

6. Lunettes selon l'une des revendications 1 à 5, **caractérisées en ce que** la gorge (13) est formée entre la portée complémentaire (11) et une saillie arrière (14) formée à l'intérieur d'un cerclage (2).

7. Lunettes selon l'une quelconque des revendications 1 à 6, **caractérisées en ce que** le bord externe (2e) d'un cerclage (2) s'étend latéralement depuis la portée complémentaire (11).

8. Lunettes selon l'une quelconque des revendications 1 à 7, **caractérisées en ce que** l'empreinte (7) présente une extension latérale (21) qui s'étend sur la géométrie arrière du bord externe (2e) du cerclage (2), la géométrie avant du bord externe (3e) de l'unité optique (3) étant, à l'état assemblé, en appui avant sur ladite extension.

9. Lunettes selon l'une quelconque des revendications 1 à 8, **caractérisées en ce que** la cavité (19) est formée dans le bord externe (2e) du cerclage (2), le bouton (18) s'étendant sur l'avant du bord externe (3e) de l'unité optique (3).

10. Lunettes selon les revendications 8 et 9, **caractérisées en ce que** la cavité (19) est formée dans l'extension latérale (21) de l'empreinte (7).

11. Lunettes selon l'une quelconque des revendications 1 à 10, **caractérisées en ce que** le bouton (18) est retirable de la cavité (19) par appui manuel sur lui.

12. Lunettes selon l'une quelconque des revendications 1 à 11, **caractérisées en ce que** le bord externe (2e) d'un cerclage (2) comprend une extrémité libre équipée d'un moyen (20, 20a) d'attache du dispositif de maintien desdites lunettes sur la tête du porteur.

13. Lunettes selon l'une quelconque des revendications 1 à 12, **caractérisées en ce que** la monture (1) comprend un pont de nez (4) qui relie les bords internes (2i) de chacun des cerclages (2).

14. Lunettes selon l'une quelconque des revendications 1 à 13, **caractérisées en ce que** chaque unité optique (3) comprend une paroi transparente (5) dont la périphérie arrière est équipée d'un joint (6) destiné à épouser le visage du porteur, le bord externe (3e) et la portée avant (10) étant formés sur ladite paroi transparente.

15. Lunettes selon l'une quelconque des revendications 1 à 13, **caractérisées en ce que** chaque unité optique (3) comprend un verre (22) dont la périphérie est équipée d'un adaptateur (23), le bord externe (3e) et la portée avant (10) étant formés sur ledit adaptateur.

16. Lunettes selon la revendication 15, **caractérisées en ce que** l'adaptateur (23) et le verre (22) présentent des moyens géométriques complémentaires (28, 29) d'association.

## Patentansprüche

1. Brille, umfassend ein Gestell (1), das mit einer Vorrichtung zum Halten der Brille auf dem Kopf eines Trägers ausgestattet ist, wobei das Gestell zwei Fassungen (2) aufweist, in denen jeweils eine optische Einheit (3) ausgelegt ist, um über eine Vorrichtung zur reversiblen Fixierung zusammengebaut zu werden, wobei jede der Vorrichtungen komplementäre geometrische Mittel umfasst, die auf einem inneren Rand (2i, 3i) von jeweils der Fassung (2) und der optischen Einheit (3) vorgesehen sind, wobei die Mittel einen Vorsprung (12) und eine Auskehlung (13) umfassen, in die der Vorsprung montiert werden kann, um eine interne Fixierung der optischen Einheit in die Fassung sicherzustellen, wobei jede der Vorrichtungen zur reversiblen Fixierung einen Knopf (18), der mit einem äußeren Rand (2e, 3e) eines der optischen Einheit (3) und der Fassung (2) fest verbunden ist, und einen Hohlraum (19), der mit einem äußeren Rand (2e, 3e) der anderen der optischen Einheit (3) und der Fassung (2) fest verbunden ist, umfasst, wobei der Knopf in den Hohlraum eingefügt werden kann, um eine äußere Verriegelung der optischen Einheit (3) in der Fassung (2) sicherzustellen, wobei die Brille **dadurch gekennzeichnet ist, dass** jede Fassung (2) eine Vertiefung (7) umfasst, die nach hinten mündet, wobei jede optische Einheit (3) eine vordere Auflagefläche (10) aufweist, die im zusammengebauten Zustand gegen eine hintere komplementäre Auflagefläche (11) der Vertiefung angeordnet ist, wobei mindestens einer der äußeren Ränder (2e, 3e) reversibel verformbar ist, um die Anordnung und die Entfernung des Knopfs in dem Hohlraum zu ermöglichen, wenn die Auflageflächen (10, 11) gegeneinander angeordnet sind.

2. Brille nach Anspruch 1, **dadurch gekennzeichnet, dass** mindestens ein äußerer Rand (2e, 3e) mit einer Haltevorrichtung (16, 17) ausgestattet ist, die sich nach hinten erstreckt, wobei die Haltevorrichtung den Knopf (18) oder den Hohlraum (19) trägt.

3. Brille nach einem der Ansprüche 1 oder 2, **dadurch gekennzeichnet, dass** die komplementären geometrischen Mittel so angeordnet sind, dass der Vorsprung (12) zunächst in der Auskehlung (13) montiert sein kann, wobei die spätere Verschiebung der Auflageflächen (10, 11) gegeneinander die äußere Verriegelung der Montage durch Anordnung des Knopfs (18) im Hohlraum (19) ermöglicht.

4. Brille nach Anspruch 3, **dadurch gekennzeichnet, dass** der Vorsprung (12) so angeordnet ist, dass er in der Auskehlung (13) durch innere Neigung der optischen Einheit (3) montiert ist und ein Scharnier für das Absenken der vorderen Auflagefläche (10) der optischen Einheit gegen die hintere Auflagefläche (11) der Fassung (2) für die äußere Verriegelung der optischen Einheit bildet.

5. Brille nach Anspruch 4, **dadurch gekennzeichnet, dass** die äußeren Ränder (2e, 3e) so angeordnet sind, dass sie, beim Absenken die elastische Verformung eines der Ränder durch Auflage auf dem anderen der Ränder sicherstellen, um die Anordnung des Knopfs (18) im Hohlraum (19) zu ermöglichen.

6. Brille nach einem der Ansprüche 1 bis 5, **dadurch gekennzeichnet, dass** die Auskehlung (13) zwischen der komplementären Auflagefläche (11) und einem hinteren Vorsprung (14), gebildet im Inneren einer Fassung (2), gebildet ist.

7. Brille nach einem der Ansprüche 1 bis 6, **dadurch gekennzeichnet, dass** sich der äußere Rand (2e) einer Fassung (2) seitlich von der komplementären Auflagefläche (11) erstreckt.

8. Brille nach einem der Ansprüche 1 bis 7, **dadurch gekennzeichnet, dass** die Vertiefung (7) eine seitliche Erweiterung (21) aufweist, die sich auf der hinteren Geometrie des äußeren Rands (2e) der Fassung (2) erstreckt, wobei die vordere Geometrie des äußeren Rands (3e) der optischen Einheit (3) im zusammengebauten Zustand auf der Erweiterung aufliegt.

9. Brille nach einem der Ansprüche 1 bis 8, **dadurch gekennzeichnet, dass** der Hohlraum (19) im äußeren Rand (2e) der Fassung (2) gebildet ist, wobei sich der Knopf (18) auf der Vorderseite des äußeren Rands (3e) der optischen Einheit (3) erstreckt.

10. Brille nach Anspruch 8 und 9, **dadurch gekennzeichnet, dass** der Hohlraum (19) in der seitlichen Erweiterung (21) der Vertiefung (7) gebildet ist.

11. Brille nach einem der Ansprüche 1 bis 10, **dadurch gekennzeichnet, dass** der Knopf (18) aus dem Hohlraum (19) durch manuelle Auflage auf ihn zurückgezogen werden kann.

12. Brille nach einem der Ansprüche 1 bis 11, **dadurch gekennzeichnet, dass** der äußere Rand (2e) einer Fassung (2) ein freies Ende umfasst, das mit einem Mittel (20, 20a) zum Befestigen der Vorrichtung zum Halten der Brille auf dem Kopf eines Trägers ausgestattet ist.

13. Brille nach einem der Ansprüche 1 bis 12, **dadurch gekennzeichnet, dass** das Gestell (1) eine Nasenbrücke (4) umfasst, die die inneren Ränder (2i) jeder der Fassungen (2) verbindet.

14. Brille nach einem der Ansprüche 1 bis 13, **dadurch gekennzeichnet, dass** jede optische Einheit (3) eine transparente Wand (5) umfasst, deren hinterer Umfang mit einer Dichtung (6) ausgestattet ist, die ausgelegt ist, um am Gesicht des Trägers anzuliegen, wobei der äußere Rand (3e) und die vordere Auflagefläche (10) auf der transparenten Wand gebildet sind.

15. Brille nach einem der Ansprüche 1 bis 13, **dadurch gekennzeichnet, dass** jede optische Einheit (3) ein Glas (22) umfasst, dessen Umfang mit einem Adapter (23) ausgestattet ist, wobei der äußere Rand (3e) und die vordere Auflagefläche (10) auf dem Adapter gebildet sind.

16. Brille nach Anspruch 15, **dadurch gekennzeichnet, dass** der Adapter (23) und das Glas (22) komplementäre geometrische Mittel (28, 29) zum Zusammenfügen aufweisen.

## Claims

1. Goggles comprising a frame (1) provided with a device for maintaining said goggles on the head of a wearer, said frame having two rims (2) in which an optical unit (3) is respectively intended for being assembled via a reversible attachment device, with each one of said devices comprising complementary geometrical means provided on an inner edge (2i, 3i) of respectively the rim (2) and the optical unit (3), said means comprising a protrusion (12) and a groove (13) wherein said protrusion is able to be mounted in order to provide an inner attachment of said optical unit in said rim, with each one of said reversible attachment devices comprising a button (18) rigidly connected to an outer edge (2e, 3e) of one among the optical unit (3) and the rim (2) and a cavity (19) rigidly connected to an outer edge (2e, 3e) of the other among the optical unit (3) and the rim (2), said button being capable of being inserted into said cavity in order to provide external locking of the optical unit (3) in the rim (2), said goggles being **characterised in that** each rim (2) has a recess (7) opening onto the rear, each optical unit (3) having a front bearing (10) which, in the assembled state, is arranged against a rear complementary bearing surface (11) of said recess, at least one of the outer edges (2e, 3e) being reversibly deformable to allow the placement and the withdrawal of said button in said cavity when the bearing surfaces (10, 11) are arranged against one another.

2. Goggles according to claim 1, **characterised in that** at least one outer edge (2e, 3e) is provided with a tab (16, 17) extending towards the rear, said tab supporting the button (18) or the cavity (19).

3. Goggles according to one of claims 1 or 2, **characterised in that** the complementary geometrical means are arranged so that the protrusion (12) can first be mounted in the groove (13), with the later displacement of the bearing surfaces (10, 11) against one another allowing for the external locking of said mounting by arrangement of the button (18) in the cavity (19).

4. Goggles according to claim 3, **characterised in that** the protrusion (12) is arranged to be mounted in the groove (13) by an internal tilting of the optical unit (3) by forming a hinge for the folding back of the front bearing surface (10) of said optical unit against the rear bearing surface (11) of the rim (2) for the purpose of the external locking of said optical unit.

5. Goggles according to claim 4, **characterised in that** the outer edges (2e, 3e) are arranged to, during the folding back, provide for the elastic deformation of one of said edges by pressing on the other of said edges in order to allow for the arrangement of the button (18) in the cavity (19).

6. Goggles according to one of claims 1 to 5, **characterised in that** the groove (13) is formed between the complementary bearing surface (11) and a rear protrusion (14) formed inside a rim (2).

7. Goggles according to any of claims 1 to 6, **characterised in that** the outer edge (2e) of a rim (2) extends laterally from the complementary bearing surface (11).

8. Goggles according to any of claims 1 to 7, **characterised in that** the recess (7) has a lateral extension (21) that extends over the rear geometry of the outer edge (2e) of the rim (2), with the front geometry of the outer edge (3e) of the optical unit (3) being, in the assembled state, pressing on the front on said extension.

9. Goggles according to any of claims 1 to 8, **characterised in that** the cavity (19) is formed in the outer edge (2e) of the rim (2), the button (18) extending over the front of the outer edge (3e) of the optical unit (3).

10. Goggles according to claims 8 and 9, **characterised in that** the cavity (19) is formed in the lateral extension (21) of the recess (7).

11. Goggles according to any of claims 1 to 10, **characterised in that** the button (18) can be withdrawn from the cavity (19) by manual pressing thereon.

12. Goggles according to any of claims 1 to 11, **characterised in that** the outer edge (2e) of a rim (2) comprises a free end provided with a means (20, 20a) of attaching the device for maintaining said goggles on the head of the wearer.

13. Goggles according to any of claims 1 to 12, **characterised in that** the frame (1) comprises a nose bridge (4) that connects the inner edges (2i) of each one of the rims (2).

14. Goggles according to any of claims 1 to 13, **characterised in that** each optical unit (3) comprises a transparent wall (5) of which the rear periphery is provided with a seal (6) intended to hug the face of the wearer, with the outer edge (3e) and the front bearing surface (10) being formed on said transparent wall.

15. Goggles according to any of claims 1 to 13, **characterised in that** each optical unit (3) comprises a glass (22) of which the periphery is provided with an adapter (23), with the outer edge (3e) and the front bearing surface (10) being formed on said adapter.

16. Goggles according to claim 15, **characterised in that** the adapter (23) and the glass (22) have complementary geometrical means (28, 29) of association.
